(19) Europäisches Patentamt
European Patent Office
Office européen des brevets

(11) Veröffentlichungsnummer: **0 117 289**
**B1**

(12) **EUROPÄISCHE PATENTSCHRIFT**

(45) Veröffentlichungstag der Patentschrift: **29.06.88**

(51) Int. Cl.⁴: **C 01 F 7/00,** A 61 K 33/10

(21) Anmeldenummer: **83111400.4**

(22) Anmeldetag: **15.11.83**

(54) **Basisches Magnesium-Aluminium-Hydroxi-Carbonat.**

(30) Priorität: **26.02.83 DE 3306822**

(43) Veröffentlichungstag der Anmeldung: **05.09.84 Patentblatt 84/36**

(45) Bekanntmachung des Hinweises auf die Patenterteilung: **29.06.88 Patentblatt 88/26**

(84) Benannte Vertragsstaaten: **AT BE CH DE FR GB IT LI LU NL SE**

(56) Entgegenhaltungen:
**FR-A-2 417 472**
**GB-A-1 185 920**
**JP-A-74 003 760**
**US-A-4 351 814**

**CHEMICAL ABSTRACTS, Band 81, Nr. 18, 4. November 1974, Nr. 106035b, Columbus, Ohio, US**

**Die Akte enthält technische Angaben, die nach dem Eingang der Anmeldung eingereicht wurden und die nicht in dieser Patentschrift enthalten sind.**

(73) Patentinhaber: **Giulini Chemie GmbH, Giulinistrasse 2, D-6700 Ludwigshafen/Rhein (DE)**

(72) Erfinder: **Schanz, Klaus, Dr. Dipl.Chem., In der Zeil 5, D-6701 Dannstadt- Schauernheim (DE)**
Erfinder: **Grund, Hartmut, Dr. Dipl.Chem., Franz-Schubert- Strasse 13, D-6701 Waldsee (DE)**
Erfinder: **Schwind, Albert, Dr. Dipl.Chem., Heinrich- Bart- Strasse 27, D-6703 Bad Dürkheim (DE)**
Erfinder: **Klehr, Peter, Saarlandstrasse 87, D-6700 Ludwigshafen/Rhein (DE)**

(74) Vertreter: **Benatzky, Erika, Dr., Giulinistrasse 2 Postfach 150550, D-6700 Ludwigshafen/Rh. (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

EP 0 117 289 B1

**Beschreibung**

Gegenstand der vorliegenden Erfindung ist kristallines Magnesium - Aluminium - Hydroxi - Carbonat der Formel

$Al_2Mg_6(OH)_{12}(CO_3)_3 \cdot x\,H_2O$ bzw.

$Al_2O_3 \cdot 6\,MgO \cdot 3\,CO_2\,(x + 6)\,H_2O$,

in der $x \geqq 4$ ist

sowie seine Verwendung in antaciden Mitteln.

Basische Magnesium - Aluminium - Carbonate sind bekannt. So werden z. B. in der GB-A- 1 086 779 Carbonate der Formel

$Al_2O_3 \cdot x\,MgO \cdot y\,CO_2 \cdot z\,H_2O$

beschrieben, in der x 0,15 bis 1,5 sein kann, y von 0,3 bis 2,5 variiert und z nicht kleiner als 2,5 ist. Hergestellt werden diese Verbindungen durch Umsetzung von Magnesiumcarbonat, Magnesiumbicarbonat oder einem Gemisch der beiden Carbonate mit einer wäßrigen Aluminiumsalzlösung, wobei wesentlich ist, daß die Magnesiumionen in Mengen anwesend sind, die oberhalb der stöchiometrisch erforderlichen Menge liegen. Auf 1 Äquivalent Aluminiumionen werden jeweils 1,05 bis 1,5 oder mehr äquivalente Magnesiumionen eingesetzt. Die basischen Carbonate werden nach der Ausfällung abfiltriert, gewaschen und getrocknet. Sie enthalten nicht mehr als 0,05 Mol- % Natrium und sind als Antacidum geeignet.

Aufgabe der vorliegenden Erfindung ist es nun, ein basisches Magnesium - Aluminium - Carbonat zu finden, das gegenüber den vorbekannten basischen Carbonaten erhöhte oder mindestens die gleiche antacide Wirksamkeit und Wirksamkeitsdauer zeigt und insbesondere kostengünstiger herstellbar ist.

Die neue Verbindung $Al_2\,Mg_6\,(OH)_{12}(CO_3)_3 \cdot xH_2O$ wird durch ein hydrothermales Verfahren hergestellt, das dadurch gekennzeichnet ist, daß Aluminiumhydroxidgel, Magnesiumhydroxid, das ganz oder teilweise durch MgO ersetzt sein kann, und basisches Magnesiumcarbonat umgesetzt und das Reaktionsprodukt durch Sprühtrocknung aus der Suspension gewonnen wird. Die Umsetzungstemperatur liegt zwischen 50 und 100° C, vorzugsweise zwischen 70 und 85° C. Die Reaktion setzt bei erhöhter Temperatur rasch ein, und zwar unter geringer Viskositätszunahme. Katalysatoren oder Beschleuniger sind nicht erforderlich.

In der US-A-4 351 814 sind Hydrotalcite mit der allgemeinen Formel

$$Mg_{1-x_1}\,M_{x1}^{3+}\,(OH)_{2+x_1-ny}\;.\;Ay^{n-}\;.m_1H_2O$$

beschrieben, in der
$0 < x_1 \leqq 0{,}6$
$0 < y \leqq 0{,}5$
$0 < m_1 \leqq 2$
sind. Diese Verbindungen zeigen eine hexagonal nadelförmige bis hexagonal faserförmige Kristallstruktur und sind wie anorganische Fasern als Füllstoffe zur Verbesserung der mechanischen Eigenschaften von Kunststoffen besonders geeignet. Sie können nach dem neuen Verfahren nicht hergestellt werden.

Gegenstand der JP-A-7 403 760 sind amphotere Metalloxidkomplexe, die durch Brennen von Verbindungen der allgemeinen Formel

$Mg_xAl_y\,(OH)_{2x+3y-2z}(OH_3)_z.aH_2O$

bei Temperaturen von 300 bis 800° C entstehen und die als Katalysatoren und Adsorbentien Verwendung finden.

In der GB-A-1 185 920 werden schließlich noch Hydrotalcite durch Umsetzung einer wäßrigen Aufschlämmung von $Al\,(OH)_3$ mit neutralem oder basischem Magnesiumcarbonat, insbesondere jedoch durch Umsetzung mit Alkalicarbonat oder Alkalibicarbonat und Magnesiumoxid oder Magnesiumhydroxid, gewonnen. Die Umsetzung von Aluminiumhydroxidgel mit Magnesiumhydroxid, das ganz oder teilweise durch Magnesiumoxid ersetzt sein kann, und basischem Magnesiumcarbonat ist nicht Gegenstand der GB-A-1 185 920.

Die Vorteile des neuen Verfahrens werden unter anderem darin gesehen, daß ein 100 %-iger Umsatz erzielt wird, ein kristallines Produkt erhalten wird, das keine Verunreinigungen enthält und die durch hydrothermale Umsetzung gewonnene Suspension unmittelbar nach Reaktionsende der Sprühtrocknung unterworfen werden kann.

Die Sprühtrocknung kann bei Eintrittstemperaturen von 275 bis 310° C und Austrittstemperaturen von 105 bis 110° C im Sprühtrockner durchgeführt werden, jedoch können diese Temperaturbereiche auch nach beiden Seiten überschritten werden.

An dieser Stelle ist erwähnenswert, daß als Aluminiumhydroxid ein aktives Aluminiumhydroxid eingesetzt wird, ein feinteiliges, amorphes, in verdünnter Säure schnellösliches Aluminiumhydroxidgel. Ein derartiges Aluminiumhydroxid kann z. B. hergestellt werden durch Fällung aus Aluminiumsalzlösungen mittels Basen, insbesondere Alkalicarbonaten. Auch die Magnesiumverbindungen sollten bei dem neuen Verfahren in aktiver Form vorliegen. Totgebranntes MgO ist beispielsweise völlig ungeeignet. Die Herstellung aktiver Magnesiumoxide und basischer Magnesiumcarbonate ist bekannt. Wird Aluminiumhydroxid mit einer Mischung aus einem basischen Magnesiumcarbonat und Magnesiumoxid umgesetzt, so sollte der Magnesiumoxidgehalt zwischen 44 und 70 Gew.-% im basischen Magnesiumcarbonat liegen.

Die nachstehende Tabelle gibt die Werte des neuen basischen Magnesium-Aluminium-Carbonats ($Al_2Mg_6(OH)_{12}(CO_3)_3 \cdot xH_2O$) und die Werte des natürlich vorkommenden $Al_2Mg_6(OH)_{16}CO_3 \cdot 4\ H_2O$ (Hydrotalcit) wieder, die im Röntgenstrahlenbeugungsspektrum erhalten worden sind. Die Linien 749,5; 577,7; 377,9; 290,6; 257,3; 255,5 und 254,3 sind im Spektrum des natürlichen Hydrotalcits nicht enthalten.

**Tabelle**

| Hydrotalcit ASTM CARD | | Verbindung gem. Erfindung | |
|---|---|---|---|
| d (pm) | d (A°) | d (pm) | d (A°) |
| - | - | - | - |
| 769 | (7,69 ) | - | - |
| - | - | - | - |
| - | - | 749,5 | (7,495) |
| - | - | 577,7 | (5,777) |
| 388 | (3,88 ) | - | - |
| - | - | - | - |
| - | - | 377,9 | (3,779) |
| - | - | 290,6 | (2,906) |
| 258 | (2,58 ) | 258,8 | (2,588) |
| - | - | 257,3 | (2,573) |
| - | - | 255,5 | (2,555) |
| - | - | 254,3 | (2,543) |
| 230 | (2,30) | 229,2 | (2,292) |
| 196 | (1,96) | 193,6 | (1,936) |
| 153 | (1,53) | 151,9 | (1,519) |
| 150 | (1,50) | 148,9 | (1,489) |

Bei der neuen Verbindung kann selbstverständlich das Kristallwasser ganz oder teilweise durch thermische Behandlung entfernt werden.

Anhand der nachstehenden Beispiele wird der Gegenstand der Erfindung nunmehr noch näher erläutert.

**Beispiel 1**

In 13 Liter Wasser werden 0,578 kg einer Magnesiumhydroxid-Paste mit einem MgO-Gehalt von 21,0 Gew.-%, 4 kg einer Aluminiumhydroxidgel-Paste mit einem $Al_2O_3$-Gehalt von 10,25 Gew.-% (3,8 Gew.-% $CO_2$) und 2,002 kg basisches Magnesiumcarbonat mit einem MgO-Gehalt von 42,3 Gew.-% und einem $CO_2$-Gehalt von 37,0 Gew.-% suspendiert. Die Suspension wird unter Rühren 6 Stunden auf 85° C erhitzt.

Das Volumen der Suspension betrug nach 6 Stunden 19 Liter und der Feststoffgehalt bei 80° C 13,1 Gew.-%. Die Viskosität der Suspension, gemessen im Brookfield RV, Spindel III, betrug

bei
10 Umdrehungen 100 mPa s ( 1.000 cp)
20 Umdrehungen 575 mPa s ( 575 cp)
50 Umdrehungen 286 mPa s ( 286 cp)
100 Umdrehungen 170 mPa s ( 170 cp.)

Nach der Sprühtrocknung lagen 2,73 kg des neuen Produktes vor, was einer Ausbeute von 100 % entspricht.

| Analyse: | prakt. | theor. |
|---|---|---|
| $Al_2O_3$ | 15,1 % | 15,54 % |
| MgO | 35,9 % | 36,87 % |
| $CO_2$ | 19,9 % | 20,12 % |

In den nachstehenden Beispielen wird anstelle von $Mg(OH)_2$ Magnesiumoxid eingesetzt.

**Beispiel 2**

In 20 Liter Wasser werden 6,838 kg Aluminiumhydroxidgel-Paste und 1,534 kg basisches Magnesiumcarbonat suspendiert. Der $Al_2O_3$-Gehalt der Paste beträgt 8,95 Gew.-% und der $CO_2$-Gehalt 3,3 Gew.-%. Das basische Magnesiumcarbonat enthält 42 Gew.-% MgO und 37,0 % $CO_2$.

Anschließend werden 0,835 kg eines im Handel erhältlichen aktiven Magnesiumoxids in die Suspension eingetragen, so daß der Anteil an MgO als basisches Magnesiumcarbonat bei 44,4 Gew.-% liegt. Nach indirekter Aufheizung mit Dampf auf 85°C (1 Stunde) erfolgt die Umsetzung in 1 Stunde bei 85°C unter intensivem Rühren. Das Endvolumen liegt bei 26 bis 27 Liter.

Bei der Sprühtrocknung der Suspension beträgt die Temperatur am Eingang 280 - 290° C und die Temperatur am Ausgang 105 - 107° C.

Die Analyse des sprühgetrockneten Produktes ergibt einen $Al_2O_3$-Gehalt von 14,9 Gew.-%, einen Magnesiumoxidgehalt von 35,6 und einen $CO_2$-Gehalt von 17,6 Gew.-%. Die Linien des Röntgenstrahlenbeugungsspektrums sind in der Tabelle angegeben.

**Beispiel 3**

Wie im Beispiel 2 werden in 20 Liter Wasser Aluminiumhydroxidgel-Paste (6,838 kg) und basisches Magnesiumcarbonat (2,140 kg) suspendiert.

Die zugesetzte Menge an Magnesiumoxid beträgt 0,571 kg, so daß der Anteil an MgO als basisches Magnesium-Carbonat 62 Gew.-% beträgt.

Wie im Beispiel 1 wird die Temperatur der Suspension mittels Dampfmantelheizung auf 85°C gebracht. Nach einer Reaktionszeit von 2 Stunden bei dieser Temperatur und Sprühtrocknung des Reaktionsproduktes wie im Beispiel 2 liegt ein Produkt vor, dessen $Al_2O_3$-Gehalt bei 14,8 Gew.-% liegt. Der MgO-Gehalt liegt bei 35,6 Gew.-% und der $CO_2$-Gehalt bei 18,1 Gew.-%.

**Beispiel 4**

Der Anteil Magnesiumoxid in Form von basischem Magnesiumcarbonat wird weiterhin erhöht, und zwar auf 90 Gew.-%.

Vorgelegt werden 18 Liter wasser, in das man 150 g MgO, das 12 Stunden durch Lagerung in 2 Liter Wasser hydratisiert worden ist, einrührt. Aschließend werden 5,564 kg Aluminiumhydroxidgel-Paste mit einem $Al_2O_3$-Gehalt von 11 % und einem $CO_2$-Gehalt von 4,1 % sowie 3,110 kg basisches Magnesiumcarbonat eingerührt. Der Magnesiumoxidgehalt beträgt wie in den Beispielen 2 und 3 42 Gew.-% und der $CO_2$-Gehalt 37,0 % $CO_2$.

Nach Aufheizung der Suspension auf 85°C wird bei dieser Temperatur noch 12 Stunden gerührt. Die Umsetzung verläuft - im Gegensatz zu den Umsetzungen in den Beispielen 2 und 3 - sehr langsam. Es wird vermutet, daß sich unter den angegebenen Versuchsbedingungen durch Alterung zunächst unlösliches, jedoch feinverteiltes Aluminiumhydroxidgel bildet, das sich unter Abspaltung von überschüssiger Kohlensäure langsam in das säurelösliche basische Magnesium - Aluminium - Hydroxi - Carbonat umwandelt. Wie in den Beispielen 2 und 3 wird die Suspension sprühgetrochnet. Der $CO_2$-Gehalt des Sprühproduktes liegt bei 18,8 Gew.-%.

Vorstehende Ergebnisse zeigen, daß die Umsetzungsgeschwindigkeit von der Reaktionsfähigkeit der Komponenten abhängt. Die Reaktionsgeschwindigkeit kann z. B. durch Art und Menge des zugesetzten Magnesiumoxids und/oder Magnesiumhydroxids beträchtlich beeinflußt werden.

Die Säurebindungskapazität der in den Beispielen hergestellten Magnesium - Aluminium - Hydroxi-Carbonate wird gemäß Sjoegren-Test bestimmt, bei dem die Säurebindungsfähigkeit pro g suspendiertem Antacidum in Abhängigkeit von der Zeit unter pH-Wert-Konstanthaltung (pH = 3) mit 0,1 n HCl/g gemessen wird.

**Beispiele**

| Beispiele | Verbrauch in 0,1 n HCl (ml) | | |
|---|---|---|---|
| | nach 5 Min. | nach 10 Min. | nach 30 Min. |
| 1 | 115 | 137 | 187 |
| 2 | 128 | 150 | 202 |
| 3 | 128 | 150 | 198 |
| 4 | 105 | 110 | 136 |

Die Säurebindungskapazität der einzelnen Magnesium - Aluminium - Hydroxi - Carbonate beträgt:

| Versuch | Säurebindungskapazität in ml 0,1 n HCl / g |
|---|---|
| 1 | 256 |
| 2 | 256 |
| 3 | 257 |
| 4 | 258 |

**Patentansprüche**

1. Kristallines, basisches Aluminium-Magnesium-Carbonat der Formel

$Al_2 Mg_6(OH)_{12}(CO_3)_3.xH_2O$,

in der $x \geqq 4$ ist,
erhältlich durch hydrothermales Umsetzen von Aluminiumhydroxid mit Magnesiumhydroxid oder Magnesiumoxid in Gegenwart von Carbonationen in stöchiometrischen Mengen bei einer Temperatur von 50 bis 100° C, dadurch gekennzeichnet, daß Aluminiumhydroxidgel, basisches Magnesiumcarbonat und Magnesiumhydroxid, das ganz oder teilweise durch Magnesiumoxid ersetzt sein kann unter Einwirkung von Scherkräften Umgesetzt und das Reaktionsprodukt durch Sprühtrocknung aus der Suspension gewonnen wird.

2. Verwendung des kristallinen, basischen Aluminium-Magnesium-Carbonats nach Anspruch 1 in antaciden Mitteln.

**Claims**

1. Crystalline, basic aluminium-magnesium-carbonate of the formula

$Al_2Mg_6(OH)_{12}(CO_3)_3.xH_2O$

in which $x \geqq 4$,
obtainable by hydrothermal reaction of aluminium hydroxide with magnesium hydroxide or magnesium oxide in the presence of carbonate ions in stoichiometric amounts at a temperature of 50 to 100°C, characterised in that aluminium hydroxide gel, basic magnesium carbonate and magnesium hydroxide, which can be replaced wholly or in part by magnesium oxide, is reacted under the action of shearing forces and the reaction product is obtained from the suspension by spray drying.

2. Use of crystalline, basic aluminium-magnesium-carbonates according to claim 1 in antacid remedies.

**Revendications**

1. Carbonate d'aluminium-magnésium basique, cristallin, de formule

$Al_2Mg(OH)_{12}(CO_3)_3 \,.\, x\, H_2O$

dans laquelle $x \geqq 4$,
obtenu par réaction hydrothermique d'hydroxyde d'aluminium avec de l'hydroxyde de magnésium ou de l'oxyde de magnésium en présence d'ions carbonate, en quantités stoechiométriques et à une température de

50 à 100 °C, caractérisé en ce qu'on fait réagir ensemble un gel d'hydroxyde d'aluminium, du carbonate de magnésium basique et de l'hydroxyde de magnésium, qui peut être complètement ou partiellement remplacé par de l'oxyde de magnesium, sous l'influence de forces de cisaillement et en ce qu'on obtient le produit de réaction par séchage par pulvérisation à partir de la suspension.

2. Application du carbonate d'aluminium-magnésium basique, cristallin, de la revendication 1 dans des produits anti-acides.